(19) Europäisches Patentamt
European Patent Office
Office européen des brevets

(11) Veröffentlichungsnummer: **0 373 494 B1**

(12) **EUROPÄISCHE PATENTSCHRIFT**

(45) Veröffentlichungstag der Patentschrift: **04.08.93**

(51) Int. Cl.$^5$: **C08F 8/44**

(21) Anmeldenummer: **89122504.7**

(22) Anmeldetag: **06.12.89**

# (54) Polyacrylsäureester mit quaternären Ammoniumgruppen.

(30) Priorität: **15.12.88 DE 3842202**

(43) Veröffentlichungstag der Anmeldung:
**20.06.90 Patentblatt 90/25**

(45) Bekanntmachung des Hinweises auf die Patenterteilung:
**04.08.93 Patentblatt 93/31**

(84) Benannte Vertragsstaaten:
**BE DE ES FR GB IT NL**

(56) Entgegenhaltungen:
**NL-C- 91 808**
**US-A- 2 435 777**
**US-A- 2 915 481**

(73) Patentinhaber: **Th. Goldschmidt AG**
**Goldschmidtstrasse 100**
**W-4300 Essen 1(DE)**

(72) Erfinder: **Fock, Jürgen, Dr.**
**Mörsenbroicher Weg 114**
**W-4000 Düsseldorf 30(DE)**
Erfinder: **Schaefer, Dietmar, Dr.**
**Milchstrasse 40**
**W-4300 Essen 14(DE)**
Erfinder: **Esselborn, Eberhard**
**Pilotystrasse 21**
**W-4300 Essen 1(DE)**

**EP 0 373 494 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Polyacrylsäureester eines mittleren Molekulargewichtes von etwa 1000 bis 50 000 mit quaternären Ammoniumgruppen und deren Verwendung in kosmetischen Zubereitungen, insbesondere in Zubereitungen zur Pflege der Haare und als Textilhilfsmittel, insbesondere zur antistatischen Ausrüstung von Textilfasern und flächigen textilen Produkten.

Die Erfindung betrifft ferner Polyacrylsäureester mit tertiären Aminogruppen als Zwischenprodukte bei der Herstellung der Polyacrylsäureester mit quaternären Ammoniumgruppen.

Polyacrylsäureester mit quaternären Ammoniumgruppen sind bekannt und werden als kationische Hilfsmittel, insbesondere zur Flokkulation von Feststoffteilchen in Schmutzwässern (EP-OS 176 757), zur antistatischen Ausrüstung von Textilfasern (DE-OS 22 42 914), zur Herstellung elektrisch leitender Kopierpapiere oder als Haarfestiger (DE-OS 24 23 182) verwendet.

Die bekannten Polyacrylsäureester mit quaternären Ammoniumgruppen werden entsprechend dem Stand der Technik durch Copolymerisation von Acrylsäureestern, insbesondere Acrylsäuremethylestern und Acrylsäureesterderivaten, welche quaternäre Ammoniumgruppen aufweisen, hergestellt.

Als Acrylsäureesterderivate mit quaternären Ammoniumgruppen werden Verbindungen, wie (Meth)-acryloyloxyethyl- oder (Meth)acryloyloxypropyltrialkyl- bzw. -dialkylbenzyl-Ammoniumhalogenide verwendet, wobei die Alkylreste insbesondere 1 bis 3 Kohlenstoffatome aufweisen.

Zusätzlich können weitere Monomere, wie Styrol, Methyl-, Ethyl-, Butyl-, Dodecyl(meth)acrylat, Vinylacetat, Vinylpropionat, N-Vinylpyrrolidon, Acrylamid, Acrylnitril, copolymerisiert werden.

Bei der radikalischen Copolymerisation der vorgenannten Monomeren entstehen Polymere mit einer sehr weiten Molekulargewichtsverteilung. Die Molekulargewichtsverteilungskurve verläuft somit relativ flach und weist überdies zwei oder mehr Maxima auf, die darauf hindeuten, daß das Polymerisationsprodukt verhältnismäßig heterogen ist. Man kann annehmen, daß der Grund hierfür in den erheblich vom Quotienten 1 abweichenden Copolymerisationsparametern der einzelnen Monomeren zu finden und insbesondere durch den ionischen Charakter der Monomeren mit quaternären Ammoniumgruppen bedingt ist.

Dabei hat sich gezeigt, daß in den durch Copolymerisation erhaltenen Polymerisaten Anteile enthalten sind, die physiologisch bedenklich sind und auch toxische Eigenschaften haben können. Es ist dabei zu vermuten, daß diese unerwünschten Eigenschaften den niedermolekularen Anteilen des Polymerisates zuzuordnen sind. Eine Abtrennung dieser Anteile aus dem Polymerisat ist in wirtschaftlicher Weise nicht möglich. Für viele Einsatzzwecke, insbesondere in der Kosmetik, ist aber die physiologische Unbedenklichkeit von Produkten zwingende Voraussetzung für ihre Verwendbarkeit. Es ist deshalb von besonderem Interesse, Polyacrylsäureester mit quaternären Ammoniumgruppen herzustellen, wobei die Polymerisate frei von physiologisch bedenklichen Bestandteilen sind.

Die Anordnung eines anionischen Polymerisationsverfahrens, wie es in der DE-OS 22 62 588 beschrieben ist, zur Herstellung von Acrylpolymeren mit einem Molekulargewicht von 500 bis 5000 stellt keine brauchbare Lösung der vorgenannten Aufgabe dar. Die Gefahr der Vergelung der Reaktionsprodukte ist hoch. Darüber hinaus ist die Überführung eines anionischen Polymerisationsverfahrens in den Betriebsmaßstab auch aus sicherheitstechnischen Gründen recht problematisch.

Aus der US-A-2 915 481 ist es bekannt, Polymethyl(meth)acrylatharze in Form eines Granulates oder Pulvers mit Mono- oder Dialkanolaminen zu erhitzen, um durch eine Umesterungsreaktion auf der Oberfläche der Harzpartikel primäre oder sekundäre Aminogruppen zu erzeugen, die es gestatten, die Harzgranulate oder Harzpulver zur Entfernung saurer Materialien, wie Mercaptane oder Hydrogensulfide, aus Gasgemischen, Erdölfraktionen oder dergleichen, zu verwenden. Es handelt sich bei den Verfahrensprodukten somit um reversibel beladbare Austauscherharze.

Der Erfindung liegt die Aufgabe zugrunde, Polyacrylsäureester mit quaternären Ammoniumgruppen herzustellen, wobei die Polymerisate möglichst geringe Anteile an niedermolekularen Produkten enthalten und physiologisch unbedenklich sein sollen.

Es wurde gefunden, daß Polymerisate, welche nur geringe Anteile an niedermolekularen Produkten enthalten, durch Umesterung geeigneter Polyacrylsäureester mit ausgewählten Aminoalkoholen erhalten werden können. Dabei werden als Zwischenprodukte Acrylsäureester mit tertiären Aminogruppen erhalten, die durch Quaternierung in die gewünschten Polyacrylsäureester mit quaternären Ammoniumgruppen überführt werden können.

Gegenstand der Erfindung sind deshalb Polyacrylsäureester eines mittleren Molekulargewichtes von etwa 1000 bis 50 000 mit quaternären Ammoniumgruppen, erhältlich durch Umesterung von durch radikalische Polymerisation erhaltenen Polyacrylsäurealkylestern, deren Alkylreste 1 bis 8 Kohlenstoffatome aufweisen, mit Verbindungen der allgemeinen Formel

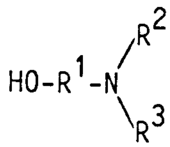

I

$R^1$ = zweiwertiger Rest der allgemeinen Formel

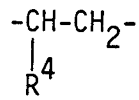

oder $-(C_nH_{2n}O)_m-C_pH_{2p}-$

$R^4$ = Wasserstoff- oder Alkylrest mit 1 bis 16 Kohlenstoffatomen,

n = 2, 3 oder 4,

m = 1 bis 20,

p = 2, 3 oder 4,

$R^2$, $R^3$ = jeweils Alkylrest mit 1 bis 18 Kohlenstoffatomen,

in solchen Mengen, daß bis zu 70 % der Estergruppen umgeestert werden, in Gegenwart eines an sich bekannten Umesterungskatalysators, bei Temperaturen von 70 bis 140° C, gegebenenfalls in Gegenwart eines Lösungsmittels, wobei als Zwischenprodukt ein Polyacrylsäureester mit tertiären Aminogruppen gebildet wird, und anschließende Quaternierung des Zwischenproduktes mit Alkyl- oder Alkylarylhalogeniden der allgemeinen Formel

$R^5X$

$R^5$ = Alkylrest mit 1 bis 4 Kohlenstoffatomen oder Benzylrest,

X = Halogenatom,

oder mit Dimethyl- oder Diethylsulfat in an sich bekannter Weise bei Temperaturen von 20 bis 140° C und gegebenenfalls erhöhtem Druck.

Im Aminoalkohol der Formel I ist $R^1$ ein zweiwertiger Rest der Formel

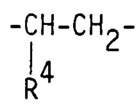

oder $-(C_nH_{2n}O)_m-C_pH_{2p}-$

Beispiele solcher Reste sind $-CH_2-CH_2-$;

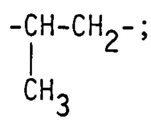
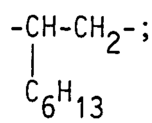

$-(CH_2)_2O(CH_2)_2-$;

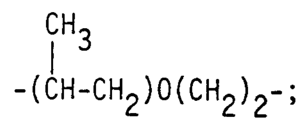

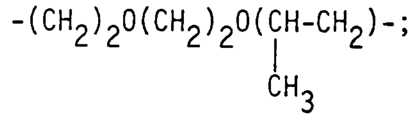 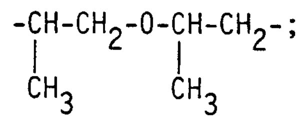

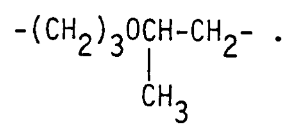

Besonders bevorzugt sind Reste, bei denen $R^4$ ein Wasserstoff-, Methyl- oder Ethylrest ist. Bei den Etherresten sind solche bevorzugt, bei denen n und p einen Wert von 2 oder 3 haben. m hat vorzugsweise einen Wert von 1 bis 10.

$R^2$ und $R^3$ sind jeweils Alkylreste mit 1 bis 18 Kohlenstoffatomen. Die Alkylreste können geradkettig oder verzweigt sein. Bevorzugt sind Alkylreste mit 1 bis 6 Kohlenstoffatomen. Insbesondere bevorzugt sind Alkylreste mit 1 bis 4 Kohlenstoffatomen, wie der Methyl-, Ethyl-, Propyl-, iso-Propyl-, Butyl- und iso-Butylrest.

Innerhalb des polymeren Moleküls können die Reste und Indices verschiedene Bedeutung bzw. Werte haben. Hierdurch bedingt können im durchschnittlichen Polymerenmolekül die Indices auch gebrochene Zahlenwerte annehmen.

Für die Umesterung werden Polyacrylsäurealkylester, deren Alkylreste 1 bis 8 Kohlenstoffatome aufweisen, eingesetzt. Vorzugsweise verwendet man Alkylester, deren Alkylrest 1 bis 4 Kohlenstoffatome enthält. Besonders bevorzugt werden deshalb die Methyl-, Ethyl- oder Butylester der Polyacrylsäure für die Umesterung ausgewählt.

Die Umesterung der Polyacrylsäureester mit den Dialkylaminoalkoholen erfolgt in an sich bekannter Weise. Sie läuft zweckmäßig in Gegenwart von an sich bekannten Umesterungskatalysatoren, wie z. B. Alkyltitanaten oder Alkalialkoholaten, ab und wird gegebenenfalls in Gegenwart eines Lösungsmittels, wie z. B. Toluol, Xylol oder Benzinfraktionen eines Siedebereiches von 80 bis 160° C durchgeführt. Das Lösungsmittel dient in erster Linie dazu, den bei der Umesterung freigesetzten Alkohol aus dem Reaktionsgemisch auszutragen. Dabei begrenzt das verwendete Lösungsmittel die Umesterungstemperaturen, die in einem Bereich von 70 bis 140° C liegen sollen.

Die Umesterungsreaktion soll dabei in solchen Mengenverhältnissen erfolgen, daß bis zu 70 %, vorzugsweise 5 bis 50 %, insbesondere bevorzugt 20 bis 50 %, der Alkylester umgeestert werden.

Als Zwischenprodukt wird dabei der Polyacrylsäureester mit tertiären Aminogruppen erhalten. Diese Verbindungen können ebenfalls im Bereich der Textilhilfsmittel und im kosmetischen Bereich, insbesondere für die Haarkosmetik eingesetzt werden.

In einem zweiten Verfahrensschritt wird das erhaltene Zwischenprodukt in ebenfalls bekannter Weise mit Alkyl- oder Alkylarylhalogeniden oder Dimethyl- bzw. Diethylethersulfat quaterniert.

Als Alkyl- oder Alkylarylhalogenide verwendet man solche der allgemeinen Formel $R^5X$, wobei $R^5$ ein Alkylrest mit 1 bis 4 Kohlenstoffatomen oder ein Benzylrest und X ein Halogenrest ist. Beispiele solcher Halogenverbindungen sind Methylchlorid, Ethylchlorid, Butylchlorid, Methylbromid oder Ethylbromid.

Die Quaternierung erfolgt bei Temperaturen von 20 bis 140° C und in Abhängigkeit vom Siedepunkt des eingesetzten Quaternierungsmittels, gegebenenfalls in einem geschlossenen System bei erhöhtem Druck.

Innerhalb des Bereiches der erfindungsgemäßen Verbindungen sind Polyacrylsäureester mit quaternären Ammoniumgruppen besonders bevorzugt, die durch Umesterung von Polyacrylsäuremethylestern mit Dimethyl- oder Diethylaminoethanol, wobei 20 bis 50 % der Estergruppen umgeestert werden, und anschließende Quaternierung mit Methylchlorid erhältlich sind.

Es ist dem Fachmann klar, daß man anstelle der Homopolymerisate auch Copolymerisate von Acrylsäureestern mit anderen polymerisierbaren Monomeren für die Umesterungsreaktion einsetzen kann.

Beispiele solcher Comonomeren sind Styrol, Methyl-, Ethyl-, Butylmethacrylat, Acrylamid, Acrylnitril.

In einer besonderen Ausgestaltungsform der Erfindung sind bis zu 50 Mol-% der Acrylsäureester durch die entsprechenden Methacrylsäureester ersetzt.

Im Gegensatz zu den nach dem Stand der Technik durch Copolymerisation von Acrylsäureestern oder Acrylsäureesterderivaten mit quaternären Ammoniumgruppen erhaltenen Copolymerisaten sind die erfindungsgemäßen Polymerisate polymer einheitlicher aufgebaut. Der Gehalt an niedermolekularen Anteilen ist ganz wesentlich vermindert. Die erfindungsgemäßen Copolymerisate haben keine toxischen oder sonstige physiologisch bedenklichen Eigenschaften.

Ein weiterer Gegenstand der Erfindung besteht in deren Verwendung in kosmetischen Zubereitungen, welche erst durch die gute Verträglichkeit der Verbindungen möglich ist. Die Polymerisate eignen sich insbesondere als Zusatzstoffe für Zubereitungen zur Pflege der Haare. Sie verbessern bereits in geringen Anwendungskonzentrationen von 0,2 bis 2 Gew.-%, bezogen auf Zubereitung, die Kämmbarkeit der Haare, insbesondere nasser Haare, und verringern deren statische Aufladung.

Eine weitere bevorzugte Verwendung der erfindungsgemäßen Verbindungen bietet sich im Textilhilfsmittelbereich an. Sie können dort als Antistatika für die Ausrüstung von Textilfasern und von Textilprodukten, wie Geweben oder Gewirken oder Vliesen, verwendet werden. Da derartig ausgerüstete Produkte mit dem menschlichen Körper in Berührung kommen, ist es auch hier von Vorteil, daß die erfindungsgemäßen Verbindungen physiologisch unbedenklich sind.

Beispiel 1

Herstellung von Polymethylacrylat durch radikalische Polymerisation (nicht erfindungsgemäß)

Eine Lösung von 0,6 g Azodiisobuttersäurenitril und 20,2 g Dodecylmercaptan in 50 g Toluol und 280 g (ca. 3,25 Mol) Methylacrylat wird innerhalb von 2 h in einen mit 53 g Toluol gefüllten Reaktor gegeben; das vorgelegte Lösungsmittel hat dabei eine Temperatur von 100° C und befindet sich unter einer Stickstoffatmosphäre. Danach werden nochmal 0,9 g Azodiisobuttersäurenitril, gelöst in 20 g Methylethylketon, innerhalb von 0,5 h nachgegeben. Schließlich wird das Reaktionsgemisch noch für 1 h bei der gleichbleibenden Temperatur von 100° C weiter erwärmt. Nach Beendigung der Reaktion wird das Lösungsmittel abdestilliert. Es verbleibt eine farblose, viskose Flüssigkeit mit einem Brechungsindex von 1,480. Aus der gelchromatographischen Untersuchung ergibt sich für das erhaltene Polymerisat ein numerisches Molekulargewicht $\overline{M}_n$ von 1950 und für das Gewichtsmittel des Molekulargewichtes $\overline{M}_w$ 3330, der Uneinheitlichkeitskoeffizient beträgt demnach 1,71. Der Restmonomerengehalt beträgt 0,1 %.

Beispiel 2 und 3

Herstellung von Polymethylacrylaten höheren Molekulargewichtes durch radikalische Polymerisation (nicht erfindungsgemäß)

Es wird verfahren wie in Beispiel 1 mit der Ausnahme, daß der Gehalt an Dodecylmercaptan gesenkt wird. In Tabelle 1 wird die Abhängigkeit der Zahlen- und Gewichtsmittel des Molekulargewichts vom Gehalt an Dodecylmercaptan gezeigt.

Tabelle 1

| Polymethylacrylat aus Beispiel | Dodecylmercaptan [Gew.-%] | Molekulargewicht $\overline{M}_n$ | Molekulargewicht $\overline{M}_w$ | Uneinheitlichkeitskoeffizient |
|---|---|---|---|---|
| 2 | 2,95 | 4 453 | 11 346 | 2,55 |
| 3 | 0,43 | 16 750 | 68 500 | 4,09 |

Beispiel 4 A

Umesterung von Polymethylacrylat mit Diethylaminoethanol

184,6 g des Polymethylacrylates aus Beispiel 1, gelöst in 185 g Toluol, werden zusammen mit 46,9 g (ca. 0,4 Mol) Diethylaminoethanol unter Stickstoff auf 120° C erhitzt. Zunächst werden möglicherweise

vorhandene Spuren von Wasser durch azeotrope Destillation entfernt. Danach erfolgt die Zugabe von 0,8 g Natriummethylat als Umesterungskatalysator. Das bei der Umesterung entstehende Methanol wird durch Fraktionierung von Toluol getrennt. Nach 2 h und nach 4 h werden jeweils 0,7 g Natriummethylat nachdosiert. Die Reaktion ist nach etwa 6 h beendet; ihr Ende wird durch eine Kopftemperatur von etwa 110° C angezeigt.

Der durch gaschromatographische Untersuchung ermittelte Anteil an Diethylaminoethanol entspricht einem Umsatz bei der Umesterung von 97,5 %; der aus dem Methanolgehalt im Destillat ermittelte Umsatz beträgt 96,2 %.

Beispiel 4 B

Quaternierung des Diethylaminoethylgruppen enthaltenden Polyacrylates

Bei dem aus Beispiel 4 A erhaltenen Produkt wird Toluol als Lösungsmittel durch Isopropanol ersetzt. 200 g des im Gewichtsverhältnis 1 : 1 mit dem Lösungsmittel verdünnten Polymerisates werden unter Stickstoff auf 110° C erhitzt und durch Einleiten von Methylchlorid ein Druck von 3,5 bis 4 bar eingestellt, der während der Reaktion durch Nachdosierung konstant gehalten wird.

Aus der Bestimmung des Chloridgehaltes und der Aminzahl ergibt sich ein Umsatz bei der Quaternierung von 98,1 bzw. 99,1 %.

Beispiele 5 A - 15 A

Umesterung von Polymethylacrylaten unterschiedlichen Molekulargewichtes mit verschiedenen Dialkylaminoalkanolen

Es wird verfahren wie in Beispiel 4 A mit der Ausnahme, daß verschiedene Dialkylaminoalkanole in, hinsichtlich der Methylestergruppen, wechselnden Molekularverhältnissen verwendet werden. Anstelle von Natriummethylat wird in einigen Fällen Isopropyltitanat als Katalysator verwendet. In Tabelle 2 werden die Reaktionsausbeuten aus der freigesetzten Menge an Methanol und aus der Aminzahl sowie die Art und die Menge des Dialkylaminoalkanols und Katalysators angegeben.

Tabelle 2

| Beispiel Nr. | Polymethyl-acrylat [$\overline{M}_n$] / Menge [g] | Dialkylamino-alkanol Art / Menge [g] | Katalysator Art / Menge [g] | Substitutionsgrad der Theorie* [%] | Umsatz aus Methanol-menge [%] | Umsatz aus Aminzahl [%] |
|---|---|---|---|---|---|---|
| 5 A | 1950 / 184,6 | DEAE / 46,9 | NAM / 2,2 | 20 | 96,2 | 97,5 |
| 6 A | 1950 / 184,6 | DEAE / 93,8 | NAM / 4,4 | 40 | 95,6 | 94,8 |
| 7 A | 1950 / 184,6 | DEAE / 140,6 | NAM / 6,5 | 60 | 92,8 | 93,4 |
| 8 A | 1950 / 184,6 | DMAE / 71,3 | NAM / 4,4 | 40 | 96,5 | 95,8 |
| 9 A | 1950 / 184,6 | DBAE / 140,6 | NAM / 4,4 | 60 | 94,3 | 94,0 |
| 10 A | 1950 / 184,6 | DMADG / 133,2 | NAM / 4,4 | 50 | 97,3 | 96,2 |
| 11 A | 1950 / 184,6 | DEAE / 93,8 | IPT / 4,0 | 40 | 97,6 | 96,8 |
| 12 A | 1950 / 184,6 | DEAE / 117,2 | IPT / 4,8 | 50 | 96,8 | 95,2 |
| 13 A | 4453 / 177,4 | DEAE / 93,8 | IPT / 4,0 | 40 | 96,5 | 95,5 |
| 14 A | 16 750 / 173,0 | DEAE / 93,8 | IPT / 4,0 | 40 | 94,6 | 93,8 |
| 15 A | 16 750 / 173,0 | DEAE / 140,6 | IPT / 5,9 | 60 | 93,2 | 91,9 |

$$* \text{ Substitutionsgrad der Theorie} = \frac{\text{Zahl der substituierten Estergruppen}}{\text{Zahl der ursprünglich vorhandenen Estergruppen}}$$

| Legende: | |
|---|---|
| | DEAE = Diethylaminoethanol |
| | DMAE = Dimethylaminoethanol |
| | DBAE = Dibutylaminoethanol |
| | DMADG = Dimethylaminodiethylenglykol |
| | NAM = Natriummethylat |
| | IPT = Isopropyltitanat |

Beispiele 5 B - 17 B

Es wird verfahren wie in Beispiel 4 B mit der Ausnahme, daß die Umesterungsprodukte 5 A - 15 A und in einigen Fällen Benzylchlorid zur Quaternierung eingesetzt werden. In Tabelle 3 werden die Ergebnisse der Quaternierung der Umesterungsprodukte aus den Beispielen 5 A - A anhand der Chlorwerte und der Aminzahlen gezeigt.

Tabelle 3

| Beispiel Nr. | Umesterungsprodukt aus Beispiel Nr. | Quaternierungsagens Art / Menge [g] | Lösungsmittel | Ausbeute (Chloridwert) [%] | Ausbeute (Aminzahl) [%] |
|---|---|---|---|---|---|
| 5 B | 5 A | MC / 9,3 | IPA | 97,7 | 99,4 |
| 6 B | 6 A | MC / 16,0 | IPA | 97,1 | 99,1 |
| 7 B | 7 A | MC / 21,2 | IPA | 97,2 | 99,5 |
| 8 B | 8 A | MC / 17,6 | IPA | 98,2 | 99,6 |
| 9 B | 8 A | BC / 44,0 | IPA | 94,2 | 98,4 |
| 10 B | 9 A | MC / 21,2 | IPA | 89,8 | 91,2 |
| 11 B | 10 A | BC / 44,3 | PD | 96,8 | 99,3 |
| 12 B | 12 A | MC / 19,6 | PD | 93,4 | 96,2 |
| 13 B | 12 A | MC / 19,6 | IPA | 92,6 | 94,3 |
| 14 B | 14 A | MC / 16,7 | PD | 92,3 | 94,9 |
| 15 B | 15 A | MC / 16,0 | IPA | 96,5 | 95,6 |
| 16 B | 15 A | MC / 16,4 | IPA | 95,2 | 97,2 |
| 17 B | 15 A | MC / 22,0 | PD | 94,6 | 96,1 |

Legende: IPA = Ipropylalkohol; PD = 1,2-Propandiol; MC = Methylchlorid; BC = Benzylchlorid

Beispiel 18

Herstellung eines Quat-Gruppen enthaltenden Polymerisates durch radikalische Copolymerisation (nicht erfindungsgemäß)

Eine Lösung von 0,6 g Azodiisobuttersäurenitril und 20,2 g Dodecylmercaptan in 93,6 g (ca. 1,09 Mol) Methylacrylat, 186,2 g (ca. 1,09 Mol) Diethylaminoethylacrylat und 50 g Toluol wird innerhalb von 2 h in einen mit 53 g Toluol gefüllten Reaktor gegeben; das vorgelegte Lösungsmittel hat dabei eine Temperatur von 100° C und befindet sich unter einer Stickstoffatmosphäre. Danach werden nochmals 0,9 g Azodiisobuttersäurenitril in 20 g Methylethylketon innerhalb von 0,5 h nachgegeben und schließlich das Reaktionsgemisch für 1 h bei der gleichbleibenden Temperatur von 100° C weiter erwärmt. Nach Beendigung der Reaktion werden Lösungsmittel und nicht umgesetzte Monomere durch Destillation entfernt.

Aus der gelchromatographischen Untersuchung ergibt sich für das erhaltene Polymerisat ein numerisches Molekulargewicht $\overline{M}_n$ von 1670 und für das Gewichtsmittel des Molekulargewichtes $\overline{M}_w$ 3720, der Uneinheitlichkeitskoeffizientbeträgt demnach 2,2. Der Restmonomerengehalt wird zu 0,1 % ermittelt.

Das erhaltene Produkt wird zunächst mit 300 g Isopropanol verdünnt und in einem Druckreaktor auf 110° C unter Stickstoff erwärmt. Dann wird soviel Methylchlorid eingeleitet, daß sich ein Druck von 3,5 bis 4 bar einstellt; dieser wird während der Reaktion durch Nachdosierung konstant gehalten. Die Reaktionsdauer beträgt 5 h.

Aus der Bestimmung des Chloridgehaltes und der Aminzahl ergibt sich ein Umsatz bei der Quaternierung von 97,9 bzw. 99,0 %.

Im Gelphasenchromatogramm zeigt sich im niedermolekularen Bereich ein Peak, der auf die Gegenwart niedermolekularer Anteile hinweist.

Anwendungstechnische Beispiele

Herstellung und Überprüfung von Haarbehandlungsmitteln unter Verwendung der in den Beispielen 13 B bzw. 6 B hergestellten Umesterungsprodukte mit Quat-Gruppen.

Konditioniershampoo:

| | | | |
|---|---|---|---|
| A | Tego® -Betain L7 [1] : | 2 | Gew.-% |
| | Antil® 141 Liquid [2] : | 3 | Gew.-% |
| B | Umesterungsprodukte aus Beispiel 13 B: | 0,5 | Gew.-% |
| C | Natriumlaurylethersulfat: | 10 | Gew.-% |
| D | Wasser: | 84,5 | Gew.-% |

Zur Herstellung des Haarbehandlungsmittels werden die Bestandteile in der aufgeführten Reihenfolge (A bis D) zusammengegeben. Jede Mischung muß vor Zugabe weiterer Komponenten klar gelöst sein.

1) Tego® -Betain L7 = Cocamidopropyl-Betain (1-Alkylamino-3-dimethyl-ammonium-propan-3-carboxymethyl-betain)

2) Antil® 141 Liquid ist ein flüssiges Verdickungsmittel auf der Basis eines nichtionogenen Fettsäurepolyalkylenglykolesters.

Cremespülung:

| | | | |
|---|---|---|---|
| A | Teginacid® X [3]: | 6 | Gew.-% |
| | Cetylalkohol: | 0,5 | Gew.-% |

| | | |
|---|---|---|
| B | Umesterungsprodukte aus Beispiel 6 B: | 1,0 Gew.-% |
| | Wasser: | 92,5 Gew.-% |

Zur Herstellung der Zubereitung werden A und B bei 70° C zusammengegeben, homogenisiert und unter Rühren abgekühlt.

3) Teginacid® X ist ein O/W-Emulgator auf der Basis einer Mischung von Glycerinmono-/-distearaten mit Polyglykolfettalkoholethern.

In der praktischen Anwendung beim halbseitigen Vergleichstest auf menschlichem Haar ergibt sich gegenüber Shampooformulierungen bzw. Cremespülungen mit polyquaternären Verbindungen aus dem Stand der Technik:

- eine höhere antistatische Wirkung
- eine nur geringfügige bzw. nicht feststellbare Beschwerung des behandelten Haares
- eine verbesserte Naßkämmbarkeit

**Patentansprüche**

**Patentansprüche für folgende Vertragsstaaten : BE, DE, FR, GB, IT, NL**

**1.** Polyacrylsäureester eines mittleren Molekulargewichtes von etwa 1.000 bis 50.000 mit quaternären Ammoniumgruppen, erhältlich durch Umesterung von durch radikalische Polymerisation erhaltenen Polyacrylsäurealkylestern, deren Alkylreste 1 bis 8 Kohlenstoffatome aufweisen, mit Verbindungen der allgemeinen Formel

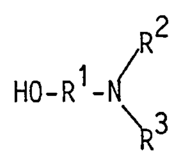

$R^1$ = zweiwertiger Rest der allgemeinen Formel

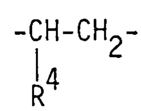

oder $-(C_nH_{2n}O)_m-C_pH_{2p}-$

$R^4$ = Wasserstoff- oder Alkylrest mit 1 bis 16 Kohlenstoffatomen,
n = 2, 3 oder 4,
m = 1 bis 20,
p = 2, 3 oder 4,
$R^2$, $R^3$ = jeweils Alkylrest mit 1 bis 18 Kohlenstoffatomen,

in solchen Mengen, daß bis zu 70 % der Estergruppen umgeestert werden, in Gegenwart eines an sich bekannten Umesterungskatalysators, bei Temperaturen von 70 bis 140° C, gegebenenfalls in Gegenwart eines Lösungsmittels, wobei als Zwischenprodukt ein Polyacrylsäureester mit tertiären Aminogruppen gebildet wird, und anschließende Quaternierung des Zwischenproduktes mit Alkyl- oder Alkylarylhalogeniden der allgemeinen Formel

$R^5X$

$R^5$ = Alkylrest mit 1 bis 4 Kohlenstoffatomen oder Benzylrest,
X = Halogenatom,
oder mit Dimethyl- oder Diethylsulfat in an sich bekannter Weise bei Temperaturen von 20 bis 140° C und gegebenenfalls erhöhtem Druck.

**2.** Polyacrylsäureester mit tertiären Aminogruppen, erhältlich als Zwischenprodukt gemäß Anspruch 1.

**3.** Polyacrylsäureester nach Anspruch 1, erhältlich durch Umesterung von Polyacrylsäuremethylestern mit Dimethyl- oder Diethylaminoethanol, wobei 20 bis 50 % der Estergruppen umgeestert werden, und anschließende Quaternierung mit Methylchlorid.

**4.** Polyacrylsäureester nach Anspruch 1, dadurch gekennzeichnet, daß bis zu 50 Mol-% der Acrylsäureester durch die entsprechenden Methacrylsäureester ersetzt sind.

**5.** Verwendung der quaternäre Ammoniumgruppen aufweisenden Polyacrylsäureester nach einem oder mehreren der vorhergehenden Ansprüche 1, 3 oder 4, in kosmetischen Zubereitungen, insbesondere zur Pflege der Haare.

**6.** Verwendung der quaternäre Ammoniumgruppen aufweisenden Polyacrylsäureester nach einem oder mehreren der vorhergehenden Ansprüche 1, 3 oder 4 als Antistatika für die Ausrüstung von Textilfasern oder textilen Produkten.

**Patentansprüche für folgenden Vertragsstaat : ES**

**1.** Verfahren zur Herstellung von Polyacrylsäureestern eines mittleren Molekulargewichtes von etwa 1.000 bis 50.000 mit quaternären Ammoniumgruppen, dadurch gekennzeichnet, daß man durch radikalische Polymerisation erhaltene Polyacrylsäureester, deren Alkylreste 1 bis 8 Kohlenstoffatome aufweisen, wobei bis zu 50 Mol-% der Acrylsäureester durch die entsprechenden Methacrylsäureester ersetzt sein können, zunächst mit Verbindungen der allgemeinen Formel

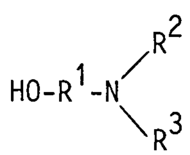

$R^1$ = zweiwertiger Rest der allgemeinen Formel

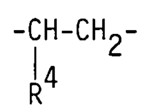

oder $-(C_nH_{2n}O)_m-C_pH_{2p}-$
$R^4$ = Wasserstoff- oder Alkylrest mit 1 bis 16 Kohlenstoffatomen,
n = 2, 3 oder 4,
m = 1 bis 20,
p = 2, 3 oder 4,
$R^2$, $R^3$ = jeweils Alkylrest mit 1 bis 18 Kohlenstoffatomen,
in solchen Mengenverhältnissen umestert, daß bis zu 70 % der Estergruppen umgeestert werden, wobei man die Reaktion in Gegenwart eines an sich bekannten Umesterungskatalysators bei Temperaturen von 70 bis 140° C, gegebenenfalls in Gegenwart eines Lösungsmittels durchführt, wobei als

Zwischenprodukt ein Polyacrylsäureester mit tertiären Aminogruppen gebildet wird, und sodann das erhaltene Zwischenprodukt mit Alkyl- oder Alkylarylhalogeniden der allgemeinen Formel

$R^5X$

$R^5$ = Alkylrest mit 1 bis 4 Kohlenstoffatomen oder Benzylrest,
X = Halogenatom,
oder mit Dimethyl- oder Diethylsulfat in an sich bekannter Weise bei Temperaturen von 20 bis 140° C und gegebenenfalls erhöhtem Druck quaterniert.

**2.** Verfahren nach Anspruch 1, dadurch gekennzeichnet, daß man Polyacrylsäuremethylester mit Dimethyl- oder Diethylaminoethanol in solchen Mengen umsetzt, daß 20 bis 50 % der Estergruppen umgeestert werden, und anschließend das erhaltene Zwischenprodukt mit Methylchlorid quaterniert.

**3.** Verwendung der nach einem Verfahren der Ansprüche 1 oder 2 hergestellten Verbindungen in kosmetischen Zubereitungen, insbesondere zur Pflege der Haare.

**4.** Verwendung der nach einem Verfahren der Ansprüche 1 oder 2 hergestellten Verbindungen als Antistatika für die Ausrüstung von Textilfasern oder textilen Produkten.

**Claims**
**Claims for the following Contracting States : BE, DE, FR, GB, IT, NL**

**1.** Polyacrylic acid esters which have an average molecular weight of about 1,000 to 50,000, contain quaternary ammonium groups and are obtainable by transesterification of polyacrylic acid alkyl esters obtained by free radical polymerisation, the alkyl radicals of which contain 1 to 8 carbon atoms, with compounds of the general formula

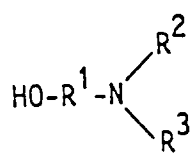

$R^1$ = a divalent radical of the general formula

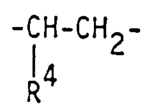

or $-(C_nH_{2n}O)_m-C_pH_{2p}-$
$R^4$ = a hydrogen radical or an alkyl radical having 1 to 16 carbon atoms,
n = 2, 3 or 4,
m = 1 to 20,
p = 2, 3 or 4,
$R^2$ and $R^3$ = in each case an alkyl radical having 1 to 18 carbon atoms,
in amounts such that up to 70 % of the ester groups are transesterified, in the presence of a transesterification catalyst which is known per se, at temperatures of 70 to 140°C, optionally in the presence of a solvent, a polyacrylic acid ester containing tertiary amino groups being formed as the intermediate product, and subsequent quaternisation of the intermediate product with alkyl or alkylaryl halides of the general formula

$R^5X$

$R^5$ = an alkyl radical having 1 to 4 carbon atoms or a benzyl radical,
X = a halogen atom,

or with dimethyl or diethyl sulphate in a manner which is known per se at temperatures of 20 to 140 °C and optionally under increased pressure.

2. Polyacrylic acid esters containing tertiary amino groups, obtainable as an intermediate product according to Claim 1.

3. Polyacrylic acid esters according to Claim 1, obtainable by transesterification of polyacrylic acid methyl esters with dimethyl- or diethylaminoethanol, 20 to 50 % of the ester groups being transesterified, and subsequent quaternisation with methyl chloride.

4. Polyacrylic acid esters according to Claim 1, characterised in that up to 50 mol % of the acrylic acid esters are replaced by the corresponding methacrylic acid esters.

5. Use of the polyacrylic acid esters containing quaternary ammonium groups according to one or more of the preceding Claims 1, 3 or 4 in cosmetic formulations, in particular for hair care.

6. Use of the polyacrylic acid esters containing quaternary ammonium groups according to one or more of the preceding Claims 1, 3 or 4 as antistatics for the treatment of textile fibres or textile products.

**Claims for the following Contracting State : ES**

1. Process for the preparation of polyacrylic acid esters which have an average molecular weight of about 1,000 to 50,000 and contain quaternary ammonium groups, characterised in that polyacrylic acid esters obtained by free radical polymerisation, the alkyl radicals of which contain 1 to 8 carbon atoms, it being possible for up to 50 mol % of the acrylic acid esters to be replaced by the corresponding methacrylic acid esters, are first transesterified with compounds of the general formula

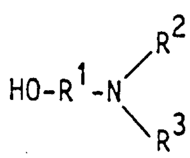

$R^1$ = a divalent radical of the general formula

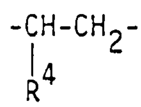

or $-(C_nH_{2n}O)_m-C_pH_{2p}-$
$R^4$ = a hydrogen radical or an alkyl radical having 1 to 16 carbon atoms,
n = 2, 3 or 4,
m = 1 to 20,
p = 2, 3 or 4,
$R^2$ and $R^3$ = in each case an alkyl radical having 1 to 18 carbon atoms,

in ratios of amounts such that up to 70 % of the ester groups are transesterified, the reaction being carried out in the presence of a transesterification catalyst which is known per se, at temperatures of 70 to 140 °C, optionally in the presence of a solvent, a polyacrylic acid ester containing tertiary amino groups being formed as the intermediate product, and the resulting intermediate product is then quaternised with alkyl or alkylaryl halides of the general formula

$R^5X$

$R^5$ = an alkyl radical having 1 to 4 carbon atoms or a benzyl radical,
X = a halogen atom,
or with dimethyl or diethyl sulphate in a manner which is known per se, at temperatures of 20 to 140 °C and optionally under increased pressure.

**2.** Process according to Claim 1, characterised in that polyacrylic acid methyl esters are reacted with dimethyl- or diethylaminoethanol in amounts such that 20 to 50 % of the ester groups are transesterified, and the resulting intermediate product is then quaternised with methyl chloride.

**3.** Use of the compounds prepared according to a process of Claims 1 or 2 in cosmetic formulations, in particular for hair care.

**4.** Use of the compounds prepared according to a process of Claims 1 or 2 as antistatics for the treatment of textile fibres or textile products.

**Revendications**
**Revendications pour les Etats contractants suivants : BE, DE, FR, GB, IT, NL,**

**1.** Polyacrylates d'un poids moléculaire moyen compris entre environ 1 000 et 50 000, contenant des groupes d'ammonium quaternaire, qu'on obtient grâce à la transestérification de polyacrylates d'alkyle obtenus par polymérisation radicalaire, dont les restes alkyles comportent de 1 à 8 atomes de carbone, avec des composés de formule générale :

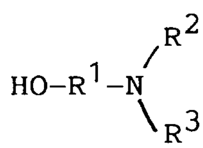

$R^1$ = reste bivalent répondant à la formule générale

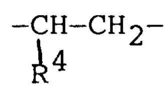

ou $-(C_nH_{2n}O)_m-C_pH_{2p}-$
$R^4$ = reste d'hydrogène ou alkyle contenant de 1 à 16 atomes de carbone,
n = 2, 3 ou 4,
m = 1 à 20,
p = 2, 3 ou 4,
$R^2$, $R^3$ = chacun un reste alkyle contenant de 1 à 18 atomes de carbone,
en des quantités telles que jusqu'à 70 % des groupes esters sont transestérifiés, en présence d'un catalyseur de transestérification connu en soi, à une température comprise entre 70 et 140 °C, éventuellement en présence d'un solvant, un polyacrylate contenant des groupes tertiaires amino étant formé, comme produit intermédiaire, et quaternisation subséquente du produit intermédiaire avec des halogénures d'alkyle ou d'alkylaryle, répondant à la formule générale

$R^5X$

$R^5$ = reste alkyle contenant de 1 à 4 atomes de carbone, ou reste benzyle,
X = atome d'halogène,
ou avec du sulfate de diméthyle ou de diéthyle, de manière connue en soi, à une température comprise entre 20 et 140 °C, et éventuellement à une pression augmentée.

**2.** Polyacrylates contenant des groupes tertiaires amino, obtenus comme produit intermédiaire selon la revendication 1.

**3.** Polyacrylates selon la revendication 1, obtenus par transestérification de polyacrylates de méthyle avec du diméthyl- ou diéthylaminoéthanol, 20 à 50 % des groupes esters étant transestérifiés, et quaternisation subséquente avec du chlorure de méthyle.

**4.** Polyacrylates selon la revendication 1, caractérisés en ce qu'on remplace jusqu'à 50 % en moles des esters de l'acide acrylique par des esters de l'acide méthacrylique.

**5.** Utilisation des polyacrylates contenant des groupes d'ammonium quaternaire selon une ou plusieurs des revendications précédentes 1, 3 ou 4, dans des préparations cosmétiques, en particulier pour les soins des cheveux.

**6.** Utilisation des polyacrylates contenant des groupes d'ammonium quaternaire selon une ou plusieurs des revendications précédentes 1, 3 ou 4, comme agents antistatiques pour l'apprêt de fibres textiles ou de produits textiles.

**Revendications pour l'Etat contractant suivant : ES**

**1.** Procédé de préparation de polyacrylates d'un poids moléculaire moyen compris entre environ 1 000 et 50 000, contenant des groupes d'ammonium quaternaire, caractérisé en ce qu'on transestérifie des polyacrylates, obtenus par polymérisation radicalaire, dont les restes alkyles comportent de 1 à 8 atomes de carbone, jusqu'à 50 % en moles des esters de l'acide acrylique pouvant être remplacés par les esters correspondants de l'acide méthacrylique, d'abord avec des composés répondant à la formule générale

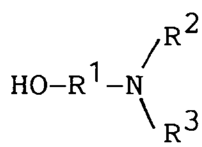

$R^1$ = reste bivalent répondant à la formule générale

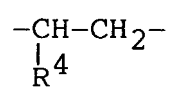

ou $-(C_nH_{2n}O)_m-C_pH_{2p}-$

$R^4$ = reste d'hydrogène ou alkyle contenant de 1 à 16 atomes de carbone,

n = 2, 3 ou 4,

m = 1 à 20,

p = 2, 3 ou 4,

$R^2$, $R^3$ = chacun un reste alkyle contenant de 1 à 18 atomes de carbone,

en des quantités telles que jusqu'à 70 % des groupes esters sont transestérifiés, la réaction étant effectuée en présence d'un catalyseur de transestérification connu en soi, à une température comprise entre 70 et 140 °C, éventuellement en présence d'un solvant, un polyacrylate contenant des groupes tertiaires amino étant formé, comme produit intermédiaire, et ensuite, on quaternise le produit intermédiaire obtenu avec des halogénures d'alkyle ou d'alkylaryle répondant à la formule générale

$R^5X$

$R^5$ = reste alkyle contenant de 1 à 4 atomes de carbone, ou reste benzyle,

X = atome d'halogène,

ou avec du sulfate de diméthyle ou de diéthyle, de manière connue en soi, à une température

comprise entre 20 et 140 °C, et éventuellement à une pression augmentée.

2. Procédé selon la revendication 1, caractérisé en ce qu'on fait réagir des polyacrylates de méthyle avec du diméthyl- ou diéthylaminoéthanol en des quantités telles que 20 à 50 % des groupes esters sont transestérifiés et ensuite, on quaternise le produit intermédiaire obtenu avec du chlorure de méthyle.

3. Utilisation des composés préparés selon un procédé des revendications 1 ou 2, dans des préparations cosmétiques, en particulier pour les soins des cheveux.

4. Utilisation des composés préparés selon un procédé des revendications 1 ou 2, comme agents antistatiques pour l'apprêt de fibres textiles ou de produits textiles.